# EUROPÄISCHE PATENTSCHRIFT

(11) **EP 1 430 832 B1**
(45) Veröffentlichungstag und Bekanntmachung des Hinweises auf die Patenterteilung: **26.04.2006**
(21) Anmeldenummer: 03024987.4
(22) Anmeldetag: 30.10.2003
(51) Int. Cl.: A61B 5/055, A61B 6/04

(54) **Patientenlagerungstisch**
Patient-supporting table
Table de support pour patient

(30) Priorität: 19.12.2002 DE 10261759
(43) Veröffentlichungstag der Anmeldung: 23.06.2004
(73) Patentinhaber: TRUMPF Medizin Systeme GmbH., 07318 Saalfeld (DE)
(72) Erfinder: Stolze, Dirk, 07318 Saalfeld (DE); Hopfe, Peter, 96337 Ludwigsstadt (DE); Georgi, Falk, 07422 Unterwirbach (DE)
(74) Vertreter: Karrais, Martin

(56) Entgegenhaltungen:
- US-A- 4 657 235
- US-A- 5 133 338
- US-A- 5 189 687
- US-A1- 2002 120 986
- US-A1- 2002 174 485
- US-B1- 6 195 578
- US-B1- 6 302 579
- US-B1- 6 416 219

## Beschreibung

Die Erfindung betrifft ein Patientenlagerungstisch mit einer eine Tischsäule aufweisenden Stützvorrichtung und einer an der Stützvorrichtung gehaltenen Tischplatte zur Lagerung eines Patienten insbesondere während der Anwendung eines therapeutischen oder diagnostischen Verfahrens, wobei die Tischplatte in Tischlängsrichtung zwischen einer bezogen auf die Stützvorrichtung ausgefahrenen Position und einer zurückgefahrenen Position hin und her verschiebbar an der Stützvorrichtung gehalten ist und wobei die Tischplatte um die Hochachse der Tischsäule zwischen einer ersten und einer zweiten Schwenkstellung hin und her verschwenkbar ist, wobei die Tischplatte in ihrer ausgefahrenen Position bezogen auf die Tischlängsrichtung einen größeren Überstand zur Stützvorrichtung aufweist als in ihrer zurückgefahrenen Position, und wobei die Stützvorrichtung eine Tischplattenaufnahme umfasst, an der die Tischplatte beweglich gehalten ist, und die Tischplattenaufnahme zur Hochachse der Tischsäule versetzt an der Tischsäule gehalten ist.

Derartige Patientenlagerungstische sind beispielsweise aus der DE 199 01 482 A1 sowie der DE 299 00 512 U1 und der korrespondierenden US 6,195,578 B1 bekannt. Sie dienen der Lagerung eines Patienten insbesondere bei Behandlungen bzw. Untersuchungen in medizinischen Therapie- und Diagnoseanlagen, beispielsweise während der Untersuchung eines Patienten mittels eines bildgebenden Verfahrens. Sie kommen insbesondere zur Lagerung eines Patienten bei Magnetresonanzanlagen und Computertomographieanlagen zum Einsatz. Zur Untersuchung des Patienten kann die Tischplatte in Längsrichtung des Tisches verschoben werden zwischen einer zurückgefahrenen Position und einer ausgefahrenen Position. In der ausgefahrenen Position steht ein stimseitiges Ende der Tischplatte in größerem Abstand zur Stützvorrichtung als in der zurückgefahrenen Position. Die Tischplatte ist außerdem um die Hochachse der Tischsäule verschwenkbar zwischen einer ersten Schwenkstellung und einer zweiten Schwenkstellung. Die erste Schwenkstellung kann hierbei zur Untersuchung des Patienten eingenommen werden, und in der zweiten Schwenkstellung kann beispielsweise der auf der Tischplatte ruhende Patient einem weiteren therapeutischen oder diagnostischen Verfahren unterzogen werden. In der zweiten Schwenkstellung kann außerdem der Transfer des Patienten vom Patientenlagerungstisch auf einen Transportwagen vorgenommen werden. Die Tischplatte ist an einer Tischplattenaufnahme beweglich gehalten, die zur Hochachse der Tischsäule versetzt an der Tischsäule gehalten ist.

Aus der US 2002/0174485 A1 ist eine Patientenlagerungsvorrichtung bekannt mit einem Patiententrolley zum An- und Abtransport eines Patienten, einer ortsfesten Stützvorrichtung und einer Tischplatte, die sowohl auf dem Trolley als auch auf der Stützvorrichtung positioniert werden kann. Mittels quer verschiebbarer Schlitten kann die Tischplatte quer zu ihrer Längsrichtung zwischen dem Trolley und der Stützvorrichtung hin- und herbewegt werden. Auf der Stützvorrichtung kann die Tischplatte in ihrer Längsrichtung verschoben werden. Der Transfer der Tischplatte vom Trolley auf die Stützvorrichtung erfolgt in einer zurückgefahrenen Stellung der Tischplatte bezogen auf die Stützvorrichtung.

Aus der DE 199 47 361 A1 ist ein Patientenlagerungstisch bekannt, bei dem die Tischplatte an einem Sockel gehalten ist. In ihrer befestigten Stellung am Sockel ist sie in Richtung ihrer Längsachse sowie in Richtung der Querachse beweglich, das heißt sie ist schwimmend gelagert.

Aufgabe der vorliegenden Erfindung ist es, einen Patientenlagerungstisch der eingangs genannten Art derart weiterzubilden, daß er einen vereinfachten Transfer des Patienten auf einen Transportwagen ermöglicht.

Diese Aufgabe wird bei einem Patientenlagerungstisch der gattungsgemäßen Art erfindungsgemäß dadurch gelöst, daß die Tischplatte in zumindest einer Schwenkstellung in ihrer ausgefahrenen Position mit der Stützvorrichtung lösbar verbindbar ist. Es hat sich gezeigt, daß durch eine derartige Ausgestaltung der Transfer des Patienten auf den Transportwagen erheblich vereinfacht werden kann. Hierzu ist zum einen vorgesehen, daß die Tischplatte mit der Stützvorrichtung lösbar verbindbar ist. Dies gibt die Möglichkeit, die Tischplatte mit dem darauf ruhenden Patienten von der Stützvorrichtung abzunehmen und auf einen Transportwagen aufzusetzen, ohne daß der Patient umgebettet werden muß. Zum anderen ist erfindungsgemäß vorgesehen, daß die Tischplatte in ihrer ausgefahrenen Position von der Stützvorrichtung lösbar und mit der Stützvorrichtung verbindbar ist. Dies hat eine erhebliche Vereinfachung des Transfers zur Folge, denn in der ausgefahrenen Position ist der Zugang zur Tischplatte für das Bedienungspersonal vereinfacht, da die Tischplatte in Längsrichtung teilweise über die Stützvorrichtung übersteht und in diesem überstehenden Bereich untergriffen werden kann.

Um das Schwenkverhalten des Patientenlagerungstisches an die jeweils herrschenden räumlichen Verhältnisse anzupassen, umfasst die Stützvorrichtung zusätzlich zur Tischsäule eine Tischplattenaufnahme, an der die Tischplatte beweglich gehalten ist, und die Tischplattenaufnahme ist zur Hochachse der Tischsäule versetzt an der Tischsäule gehalten. Die Tischplattenaufnahme kann hierbei starr mit der Tischsäule verbunden sein, alternativ kann vorgesehen sein, daß die Tischplattenaufnahme um eine quer zur Längsachse des Tisches ausgerichtete Kippachse verkippbar an der Tischsäule gehalten ist. Eine derartige Ausgestaltung hat den Vorteil, daß die beweglich an der Tischplattenaufnahme gehaltene Tischplatte schräg zur Horizontalen ausgerichtet werden kann,

Besonders günstig ist es, wenn die Tischplatte in ihrer ausgefahrenen Position um eine Länge von mindestens 1,5 m über die Stützvorrichtung übersteht. In ihrer ausgefahrenen Position ist die Tischplatte freitragend an der Stützvorrichtung gehalten, wobei sie einen Überstand von mindestens 1,5 m, vorzugsweise 1,5 m bis etwa 2,5 m aufweist. Dies ermöglicht eine besonders gute Zugänglichkeit der Tischplatte zum Transfer auf einen Transportwagen.

Vorzugsweise ist die Tischplatte über mindestens 1,5 m, vorzugsweise 1,5 bis 2,0 m in Längsrichtung verschiebbar an der Stützvorrichtung gehalten. Dies hat den Vorteil, daß der Patient über seine gesamte Körperlänge durch Verschieben der Tischplatte relativ zur Stützvorrichtung untersucht oder therapiert werden kann, beispielsweise kann der Patient über seine gesamte Körperlänge durchleuchtet werden.

Bei einer bevorzugten Ausführungsform der Erfindung ist vorgesehen, daß die Tischplatte in ihrer ausgefahrenen Position auf einen Transportwagen aufsetzbar ist. Der Transfer des Patienten auf einen Transportwagen kann durch eine derartige Ausgestaltung besonders einfach durchgeführt werden. Zur Untersuchung nimmt die Tischplatte ihre erste Schwenkstellung ein, in der sie beispielsweise mit einem darauf ruhenden Patienten in eine Magnetresonanzanlage eingeführt und anschließend wieder zurückgezogen werden kann. Daraufhin kann die Tischplatte in ihre zweite Schwenkstellung verschwenkt und erneut in ihre ausgefahrene Stellung verschoben werden. Die Tischsäule kann hierbei eine derartige Höhenposition einnehmen, daß ein Transportwagen unterhalb des über die Stützvorrichtung überstehenden Bereiches der Tischplatte positioniert werden kann. Anschließend kann die Tischsäule abgesenkt oder alternativ der die Tischplatte aufnehmende Bereich des Transportwagens angehoben werden, so daß die Tischplatte mit ihrem überstehenden Bereich auf dem Transportwagen aufsitzt, und dann kann eine Arretierung des überstehenden Bereiches der Tischplatte auf dem Transportwagen vorgenommen und die Tischplatte von der Tischsäule gelöst werden.

Eine besonders einfache Handhabung beim Transfer des Patienten kann dadurch erzielt werden, daß die Tischsäule höhenverstellbar ist.

Zur Sicherstellung der Drehbarkeit der Tischplatte um die Hochachse der Tischsäule kann vorgesehen sein, daß die Tischplatte drehfest an der Stützvorrichtung gehalten ist und daß die Stützvorrichtung um die Hochachse der Tischsäule verschwenkbar ist. Die Tischsäule kann beispielsweise an einem ortsfest gehaltenen Sockel drehbar gelagert sein.

Alternativ kann vorgesehen sein, daß die Tischplatte relativ zur Stützvorrichtung um die Hochachse der Tischsäule verschwenkbar ist.

Vorzugsweise ist die Tischplatte um mindestens 90° um die Hochachse der Tischsäule verschwenkbar. Ein derartiger Verschwenkwinkelbereich stellt sicher, daß die ihre ausgefahrene Position einnehmende Tischplatte in ihrer zweiten Schwenkstellung, beispielsweise außerhalb einer Magnetresonanzanlage, frei zugänglich ist.

Zur Erzielung einer besonders stabilen Halterung der Tischplatte an der Tischplattenaufnahme ist es von Vorteil, wenn die Tischplattenaufnahme die Tischplatte längsseitig umgreift. Hierzu kann vorgesehen sein, daß die Tischplattenaufnahme quer zur Tischlängsachse C-förmig ausgestaltet ist.

Vorzugsweise ist die gesamte Tischplatte aus einem eine hohe Transparenz für Röntgenstrahlung aufweisenden Material gefertigt.

Die Gesamtlänge der Tischplatte beträgt vorzugsweise mehr als 2,0 m, insbesondere mehr als 2,3 m. Dies stellt sicher, daß zum einen der Patient über seine gesamte Körperlänge beispielsweise mittels eines bildgebenden Verfahrens untersucht werden kann, daß aber zum anderen eine zuverlässige Halterung der Tischplatte in ihrer ausgefahrenen Position gewährleistet ist, wobei die Tischplatte über eine Länge von mindestens 2,0 m in Längsrichtung verschiebbar an der Tischsäule bzw. der Tischplattenaufnahme gehalten ist.

Die nachfolgende Beschreibung eines bevorzugten Ausführungsbeispieles der Erfindung dient im Zusammenhang mit der Zeichnung der näheren Erläuterung. Es zeigen:
- Figuren 1 und 2:: eine schematische Seitenansicht bzw. eine schematische Draufsicht eines Patientenlagerungstisches in Kombination mit einer Magnetresonanzanlage, wobei die Tischplatte des Patientenlagerungstisches ihre erste Schwenkstellung und eine ausgefahrene Position einnimmt;
- Figuren 3 und 4:: eine schematische Seitenansicht bzw. schematische Draufsicht des Patientenlagerungstisches, wobei die Tischplatte ihre erste Schwenkstellung und eine zurückgezogene Position einnimmt;
- Figuren 5 und 6:: eine schematische Seitenansicht bzw. schematische Draufsicht des Patientenlagerungstisches, wobei die Tischplatte ihre zweite Schwenkstellung und eine zurückgezogene Position einnimmt;
- Figuren 7 und 8:: eine schematische Seitenansicht bzw, schematische Draufsicht des Patientenlagerungstisches, wobei die Tischplatte ihre zweite Schwenkstellung und eine ausgefahrene Position einnimmt;
- Figur 9:: eine schematische schaubildliche Darstellung eines erfindungsgemäßen Patientenlagerungstisches, wobei die Tischplatte ihre erste Schwenkstellung und eine ausgefahrene Position einnimmt und
- Figur 10:: eine schematische schaubildliche Darstellung des erfindumgsgemäßen Patientenlagerungstisches, wobei die Tischplatte ihre zweite Schwenkstellung und eine ausgefahrene Position einnimmt.

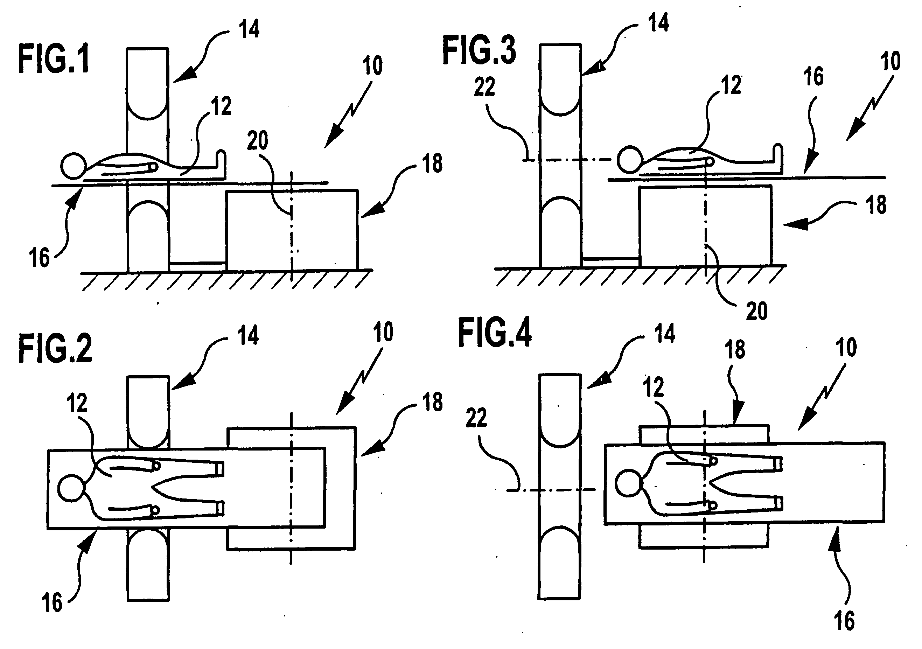

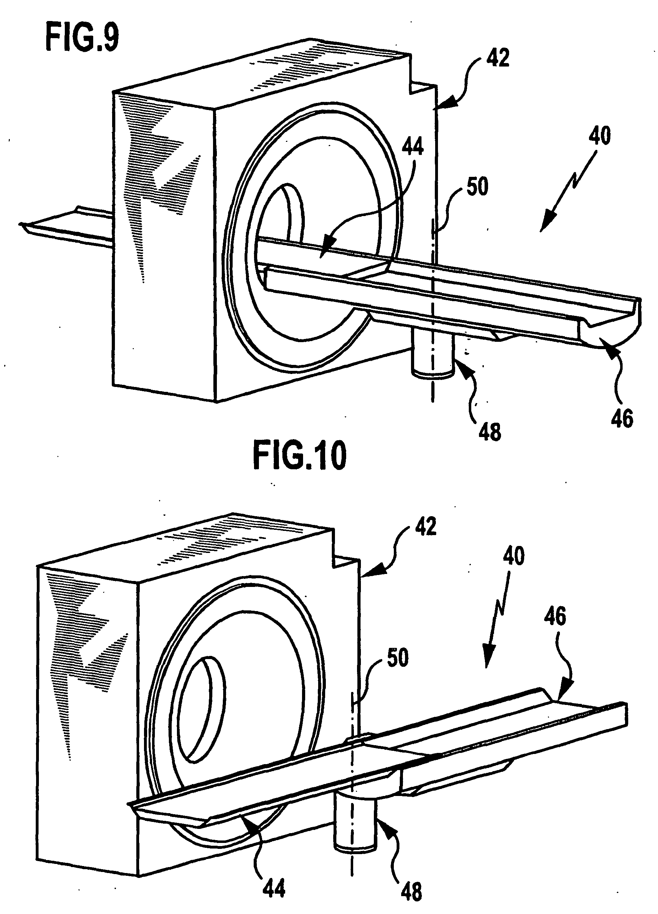

Eine bevorzugte Ausführungsform eines erfindungsgemäßen Patientenlagerungstisches in in den Figuren 9 und 10 dargestellt. Zur Erläuterung der Erfindung wird jedoch zunächst unter Bezugnahme auf die Figuren 1 bis 8 ein einfacherer Patientenlagerungstisch beschrieben, der insgesamt mit dem Bezugszeichen 10 belegt ist. Er kommt zur Untersuchung eines Patienten 12 in einer Magnetresonanzanlage 14 zum Einsatz. Der Patienfienlagerungstisch 10 umfaßt eine Tischplatte 16, auf der der Patient 12 ruht, sowie eine Stützvorrichtung in Form einer eine Tischsäule 18, an der die Tischplatte 16 in Längsrichtung der Tischplatte 16 verschiebbar gehalten ist, so daß die Tischplatte 16 zwischen einer beispielsweise in den Figuren 3 und 4 dargestellten zurückgezogenen Position und einer beispielsweise in den Figuren 1 und 2 dargestellten ausgefahrenen Position hin und her verschiebbar ist. Die zurückgezogene Position ist so gewählt, daß der Schwerpunkt des Patienten ungefähr mit der Hochachse 20 der Tischsäule 18 zusamrnenfällt. Die ausgefahrene Position der Tischplatte 16 ist derart gewählt, daß der Patient mit seiner gesamten Körperlänge über die Tischsäule 18 übersteht.

Dies gibt die Möglichkeit, den auf der Tischplatte 16 ruhenden Patienten zur zumindest teilweisen Durchleuchtung in die Magnetresonanzanlage 14 einzuführen. Hierbei können zur Verschiebung der Tischplatte 16 in die Tischsäule 18 integrierte, an sich bekannte und deshalb in der Zeichnung nicht dargestellte elektrische Stellmotoren zum Einsatz kommen, die von einer zentralen Steuereinheit der Magnetresonanzanlage 14 steuerbar sind.

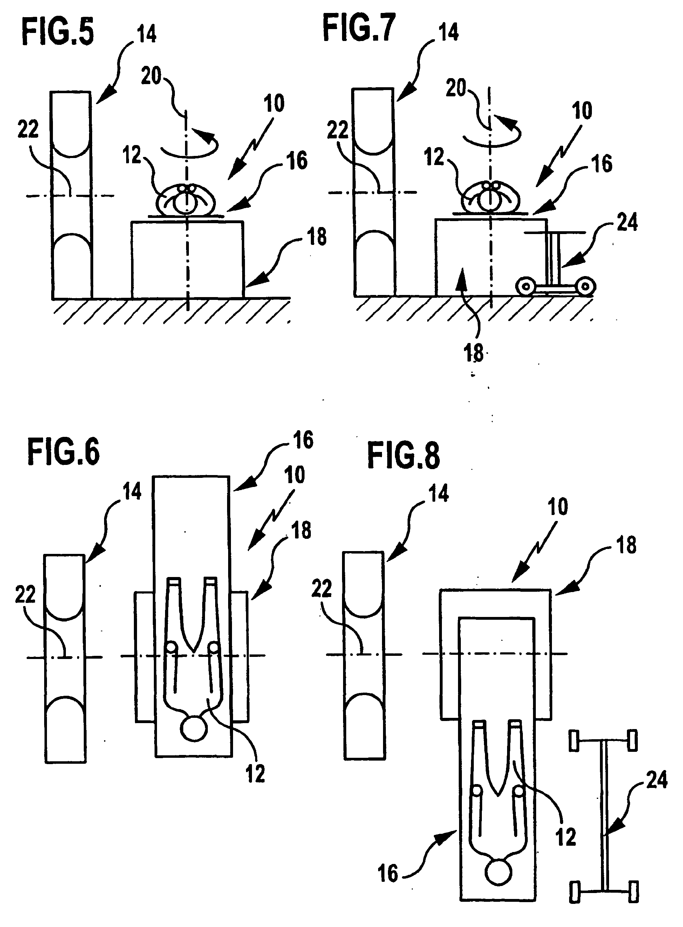

Die Tischplatte 16 kann nicht nur in Längsrichtung definiert hin und her verschoben werden, sondern sie kann auch um die Hochachse 20 der Tischsäule 18 zwischen einer ersten, in den Figuren 1 bis 4 dargestellten Schwenkstellung und einer zweiten, in den Figuren 5 bis 8 dargestellten Schwenkstellung hin und her verschwenkt werden. Die Tischplatte 16 ist drehfest an der Tischsäule 18 angeordnet und die Tischsäule 18 kann um die Hochachse 20 zwischen der ersten und der zweiten Schwenkstellung hin und her verschwenkt werden. In der ersten Schwenkstellung ist die Längsachse der Tischplatte 18 parallel zur Symmetrieachse 22 der Magnetresonanzanlage 14 ausgerichtet, und in der zweiten Schwenkstellung nimmt die Tischplatte 16 eine um 90° verschwenkte Ausrichtung ein, so daß die Tischlängsachse senkrecht zur Symmetrieachse 22 der Magnetresonanzanlage 14 ausgerichtet ist.

Auch bei Einnahme der zweiten Schwenkstellung kann die Tischplatte 16 zwischen ihrer ausgefahrenen Position und ihrer zurückgezogenen Position hin und her verschoben werden, insbesondere ist es möglich, die Tischplatte 16 in ihrer zweiten Schwenkstellung soweit relativ zur Tischsäule 18 zu verschieben, daß ein Transportwagen 24 unterhalb des über die Tischsäule 18 überstehenden Längsbereiches der Tischplatte 16 positioniert werden kann.

Die Tischsäule 18 kann in ihrer Höhe verstellt werden. Dies gibt die Möglichkeit, die Tischplatte 16 in ihrer ausgefahrenen Position, nachdem der Transportwagen 24 unterhalb des überstehenden Bereiches der Tischplatte 16 positioniert wurde, auf den Transportwagen 24 abzusetzen und am Transportwagen 24 zu arretieren. Anschließend kann die Tischplatte 16 von der Tischsäule 18 gelöst und diese soweit nach unten abgesenkt werden, daß sie die nunmehr auf den Transportwagen 24 aufsitzende Tischplatte 16 völlig freigibt, so daß die Tischplatte 16 zusammen mit dem Transportwagen 24 entfernt werden kann.

In den Figuren 9 und 10 ist schematisch eine Ausführungsform des erfindungsgemäßen Patientenlagerungstisches dargestellt und insgesamt mit dem Bezugszeichen 40 belegt. Dieser kommt wiederum zur Lagerung eines Patienten während einer Untersuchung in einer Magnetresonanzanlage 42 zum Einsatz und umfaßt eine Tischplatte 44, die über eine gesamte Körperlänge eines Patienten, also über eine Längserstreckung von mindestens 1,5 m bis etwa 2,0 m, in die Magnetresonanzanlage 42 koordiniert eingeführt werden kann. Hierzu ist die Tischplatte 44 verschiebbar an einer Tischplattenaufnahme 46 gehalten, die die Tischplatte 44 längsseitig umgreift und ihrerseits an einer seitlich angeordneten, höhenverstellbaren Tischsäule 48 gehalten ist. Die Tischptattenaufnahme 46 bildet in Kombination mit der Tischsäule 48 eine Stützvorrichtung des Patiententagerungstisches 40, an der die Tischplatte 44 gehalten ist. Die Tischplattenaufnahme 46 ist hierbei versetzt zur Hochachse 50 der Tischsäule 48 angeordnet und weist in Längsrichtung der Tischplatte 44 einen ungefähr C-förmigen Querschnitt auf.

Die Hochachse 50 der Tischsäule 48 bildet eine Drehachse für die Tischplatte 44 und die Tischplattenaufnahme 46, d. h. die Drehachse der Tischplatte 44 ist seitlich außerhalb der Tischplatte 44 positioniert. Dies wird insbesondere aus Figur 10 deutlich. Eine derartige Ausrichtung hat den vorteil, daß das Schwenkverhalten der Tischplatte 44 und der Tischplattenaufnahme 46 an die jeweils vorliegenden räumlichen Verhältnisse angepaßt werden kann.

Die Tischplatte 44 ist lösbar verbindbar an der Tischplattenaufnahme 46 gehalten und kann in ihrer um 90° zur Symmetrieachse der Magnetresonanzanlage 42 verschwenkten zweiten Schwenkstellung in ihre ausgefahrene Position verschoben und in dieser Position von der Tischplattenaufnahme 46 gelöst werden. Wie insbesondere aus Figur 10 deutlich wird, weist die in ihre zweite Schwenkstellung verschwenkte Tischplatte 44 in ihrer ausgefahrenen Position einen ganz erheblichen Überstand zur Tischplattenaufnahme 46 auf, d. h. die Tischplatte 44 ist freitragend an der Tischplatteaufnahme 46 gehalten, und mit dem überstehenden Bereich kann die Tischplatte 44 durch Absenken der höhenverstellbaren Tischsäule 48 entsprechend der voranstehend unter Bezugnahme auf die Figuren 1 bis 8 erläuterten Tischplatte 16 auf einen unterhalb des überstehenden Bereichs der Tischplatte 44 positionierten Transportwagen (in Figur 10 nicht dargestellt) aufgesetzt werden.

Aus dem voranstehenden wird deutlich, daß mittels des erfindungsgemäßen Patientenlagerungstisches der Transfer eines Patienten vom Patientenlagerungstisch auf einen Transportwagen ganz erheblich vereinfacht werden kann, wobei gleichzeitig sichergestellt ist, daß der auf der Tischplatte ruhende Patient mittels eines bildgebenden Verfahrens, beispielsweise mittels einer Magnetresonanzanlage oder einer Computertomographieanlage, untersucht werden kann.

## Patentansprüche

1. Patientenlagerungstisch mit einer eine Tischsäule (48) aufweisenden Stützvorrichtung (46, 48) und einer an der Stützvorrichtung (46, 48) gehaltenen Tischplatte (44) zur Lagerung eines Patienten insbesondere während der Anwendung eines therapeutischen oder diagnostischen Verfahrens, wobei die Tischplatte (44) in Tischlängsrichtung zwischen einer bezogen auf die Stützvorrichtung (46, 48) zurückgefahrenen und einer ausgefahrenen Position hin und her verschiebbar und um die Hochachse (20) der Tschsäule (48) zwischen einer ersten und einer zweiten Schwenkstellung hin und her verschwenkbar ist, wobei die Tischplatte (44) in ihrer ausgefahrenen Position bezogen auf die Tischlängsrichtung einen größeren Überstand zur Stützvorrichtung (46, 48) aufweist als in ihrer zurückgefahrenen Position und wobei die Stützvorrichtung eine Tischplattenaufnahme (46) umfaßt, an der die Tischplatte (44) beweglich gehalten ist, und die Tischplattenaufnahme (46) zur Hochachse (50) der Tischsäule (48) versetzt an der Tischsäule (48) gehalten ist, **dadurch gekennzeichnet, daß** die Tischplatte (16; 44) in mindestens einer Schwenkstellung in ihrer ausgefahrenen Position mit der Stützvorrichtung (18; 46, 48) lösbar verbindbar ist.

2. Patientenlagerungstisch nach Anspruch 1, **dadurch gekennzeichnet, daß** die Tischplatte (16; 44) in ihrer ausgefahrenen Position um eine Länge von mindestens 1,5 m über die Stützvorrichtung (18; 46, 48) übersteht.

3. Patienteniagerungstisch nach Anspruch 1 oder 2, **dadurch gekennzeichnet, daß** die Tischplatte (16; 44) in ihrer ausgefahrenen Position auf einen Transportwagen (24) aufsetzbar ist.

4. Patientenlagerungstisch nach Anspruch 1, 2 oder 3, **dadurch gekennzeichnet, daß** die Tischsäule (18; 48) höhenverstellbar ist.

5. Patientenlagerungstisch nach einem der voranstehenden Ansprüche, **dadurch gekennzeichnet, daß** die Tischplatte (16; 44) drehfest an der Stützvorrichtung (18; 46, 48) gehalten ist und daß die Stützvorrichtung (18; 46, 48) um die Hochachse (20; 50) der Tischsäule (18; 48) verschwenkbar ist.

6. Patientenlagerungstisch nach einem der voranstehenden Ansprüche, **dadurch gekennzeichnet, daß** die Tischplatte (16; 44) um mindestens 90° um die Hochachse (20; 50) der Tischsäule (18; 48) verschwenkbar ist.

7. Patientenlagerungstisch nach einem der voranstehenden Ansprüche, **dadurch gekennzeichnet, daß** die Tischplattenaufnahme (46) die Tischplatte (44) längsseitig umgreift.

## Claims

1. Patient supporting table with a supporting device (46, 48) comprising a table column (48) and with a table top (44) for supporting a patient, in particular, during application of a therapeutic or diagnostic procedure, the table top (44) being held on the supporting device (46, 48) and being displaceable back and forth in the longitudinal direction of the table between a retracted and an extended position in relation to the supporting device (46, 48) and being pivotable back and forth about the vertical axis (20) of the table column (48) between a first and a second pivotal position, the table top (44) having in its extended position a greater projection from the supporting device (46, 48) in relation to the longitudinal direction of the table than in its retracted position, the supporting device comprising a table top receptacle (46) on which the table top (44) is movably held, and the table top receptacle (46) being held on the table column (48) at an offset to the vertical axis (50) of the table column (48), **characterized in that** in at least one pivotal position the table top (16; 44) is detachably connectable in its extended position to the supporting device (18; 46, 48).

2. Patient supporting table in accordance with Claim 1, **characterized in that** in its extended position the table top (16; 44) projects by a length of at least 1.5 m over the supporting device (18; 46, 48).

3. Patient supporting table in accordance with Claim 1 or 2, **characterized in that** the table top (16; 44) is placeable in its extended position on a trolley (24).

4. Patient supporting table in accordance with Claim 1, 2 or 3, **characterized in that** the table column (18; 48) is height-adjustable.

5. Patient supporting table in accordance with any one of the preceding Claims, **characterized in that** the table top (16; 44) is non-rotatably held on the supporting device (18; 46, 48), and **in that** the supporting device (18; 46, 48) is pivotable about the vertical axis (20; 50) of the table column (18; 48).

6. Patient supporting table in accordance with any one of the preceding Claims, **characterized in that** the table top (16; 44) is pivotable through at least 90° about the vertical axis (20; 50) of the table column (18; 48).

7. Patient supporting table in accordance with any one of the preceding Claims, **characterized in that** the table top receptacle (46) embraces the table top (44) at the longitudinal sides thereof.

## Revendications

1. Table pour patient en position allongée, comprenant un dispositif de support (46, 48) présentant une colonne de table (48) et un plateau de table (44) maintenu sur le dispositif de support (46, 48), permettant de placer un patient en position allongée, en particulier pendant l'utilisation d'un procédé thérapeutique ou diagnostique, où le plateau (44) de la table est mobile en va-et-vient, dans la direction longitudinale de la table, entre une position rentrée et une position déployée par rapport au dispositif de support (46, 48), et peut pivoter en va-et-vient autour de l'axe vertical (20) de la colonne (48) de la table, entre une première position de pivotement et une seconde position de pivotement, où le plateau (44) de la table, dans sa position déployée, présente par rapport à la direction longitudinale de la table, un débordement (ou porte-à-faux) par rapport au dispositif de support (46, 48) plus important que dans sa position rentrée, et où le dispositif de support comprend un logement de plateau de table (46) sur lequel le plateau (44) de la table est maintenu de façon mobile, et le logement (46) du plateau de la table est maintenu sur la colonne (48) de la table, en étant décalé par rapport à l'axe vertical (50) de la colonne (48) de la table,
**caractérisée en ce que** le plateau (16 ; 44) de la table, dans au moins une position de pivotement et dans sa position déployée, peut être relié au dispositif de support (18 ; 46, 48), de manière détachable.

2. Table pour patient en position allongée selon la revendication 1, **caractérisée en ce que** le plateau (16 ; 44) de la table, dans sa position déployée, dépasse d'une longueur d'au moins 1,5 m par rapport au dispositif de support (18 ; 46, 48).

3. Table pour patient en position allongée selon la revendication 1 ou 2, **caractérisée en ce que** le plateau (16 ; 44) de la table, dans sa position déployée, peut être placé sur un chariot de transport (24).

4. Table pour patient en position allongée selon les revendications 1, 2 ou 3, **caractérisée en ce que** la colonne (18 ; 48) de la table est réglable en hauteur.

5. Table pour patient en position allongée selon l'une quelconque des revendications précédentes, **caractérisée en ce que** le plateau (16 ; 44) de la table est maintenu, fixe en rotation, sur le dispositif de support (18 ; 46, 48), et **en ce que** le dispositif de support (18 ; 46, 48) peut pivoter autour de l'axe vertical (20 ; 50) de la colonne (18 ; 48) de la table.

6. Table pour patient en position allongée selon l'une quelconque des revendications précédentes, **caractérisée en ce que** le plateau (16 ; 44) de la table peut pivoter d'au moins 90° autour de l'axe vertical (20 ; 50) de la colonne (18 ; 48) de la table.

7. Table pour patient en position allongée selon l'une quelconque des revendications précédentes, **caractérisée en ce que** le logement (46) du plateau de la table entoure, sur le côté longitudinal, le plateau (44) de la table.
